# EUROPÄISCHE PATENTSCHRIFT

(11) **EP 4 142 658 B1**
(45) Veröffentlichungstag und Bekanntmachung des Hinweises auf die Patenterteilung: **28.08.2024**
(21) Anmeldenummer: 21749192.7
(22) Anmeldetag: 26.07.2021
(51) Int. Cl.: A61F 2/74, C10M 169/04, C10N 40/08, A61F 2/50

(54) **ORTHOPÄDIETECHNISCHE EINRICHTUNG**
ORTHOPEDIC DEVICE
DISPOSITIF ORTHOPÉDIQUE

(30) Priorität: 31.07.2020 DE 102020120304
(43) Veröffentlichungstag der Anmeldung: 08.03.2023
(73) Patentinhaber: Ottobock SE & Co. KGaA, 37115 Duderstadt (DE)
(72) Erfinder: NÖRTHEMANN, Jens, 37115 Duderstadt (DE); SCHMERGLATT, André, 37136 Landolfshausen (DE)
(74) Vertreter: Gramm, Lins & Partner Patent- und Rechtsanwälte PartGmbB
(86) Internationale Anmeldenummer: PCT/EP2021/070829
(87) Internationale Veröffentlichungsnummer: WO 2022/023251

(56) Entgegenhaltungen:
- DE-A1- 2 101 303
- JP-A- 2019 136 225
- JP-A- H0 379 699
- JP-B2- H0 631 390
- US-A1- 2004 083 007

## Beschreibung

Die Erfindung betrifft eine orthopädietechnische Einrichtung mit wenigstens einem Gelenk und einem Hydrauliksystem mit wenigstens einem Ventil, wobei das Hydrauliksystem mit einer Hydraulikflüssigkeit gefüllt ist, die ein Silikonöl aufweist. Die Erfindung betrifft zudem ein Verfahren zum Füllen eines Hydrauliksystems für eine derartige orthopädietechnische Einrichtung.

Eine derartige orthopädietechnische Einrichtung ist beispielsweise aus der US 2004/0083007 A1 bekannt. Die verwendete Hydraulikflüssigkeit ist ein Silikonöl. In der JP H-03-79699 geht es um die Schmierung von sich bewegenden Komponenten, durch die ein Drehmoment übertragen werden soll. Der direkte Kontakt von Kupfer- oder Stahlbauteilen, die sich zueinander bewegen, soll durch die Schmierung verhindert werden. Es wird ein Silikonöl mit einem ester-basierten Zusatzstoff vorgeschlagen, da diese Kombination gute Hafteigenschaften an metallischen Oberflächen aufweist. Die JP H-06-31390 hingegen befasst sich mit Hydraulikölen für die Kraftfahrzeugindustrie. Dabei werden Hydrauliköle für Stoßdämpfer beschrieben, die besonders im Bereich hoher Temperaturen und Drehzahlen von mehreren 1000 pro Minute gute Viskositätseigenschaften aufweisen sollen. Es werden Silikonöle mit einem ester-basierten Zusatzstoff verwendet.

Orthopädietechnische Einrichtungen sind aus dem Stand der Technik insbesondere in Form von Prothesen oder Orthesen, aber auch in Form anderer unterstützender Einrichtungen, bekannt. Dies betrifft beispielsweise Exoskelette und andere Vorrichtungen, die beispielsweise bei anstrengenden Arbeiten, beispielsweise über Kopf, den Benutzer der Vorrichtung unterstützen, sodass die anstrengende Arbeit länger durchgeführt werden kann. Gattungsgemäße orthopädietechnische Einrichtungen verfügen über wenigstens ein Gelenk mit einem Hydrauliksystem. Dies kann beispielsweise einen hydraulischen Dämpfer beinhalten. In vielen Ausführungsformen verfügt das Hydrauliksystem über mehrere Kammern, die miteinander durch einen Kanal oder eine Leitung oder auf sonstige Weise fluidtechnisch verbunden sind. Wird nun beispielsweise das Gelenk bewegt, wird dadurch die Hydraulikflüssigkeit von der einen Kammer in eine andere Kammer befördert. Je nach Durchmesser der fluidtechnischen Verbindung zwischen den beiden betroffenen Kammern entsteht hierbei ein der Bewegung entgegenstehender Strömungswiderstand, also eine Dämpfung. In diesem Hydrauliksystem befindet sich gattungsgemäß wenigstens ein Ventil, das beispielsweise verwendet werden kann, um den genannten Strömungswiderstand zu variieren. Das Ventil kann geöffnet oder geschlossen oder teilweise geöffnet sein und so den der Strömung zur Verfügung stehenden Durchmesser, also beispielsweise die Öffnungsweite, der fluidtechnischen Verbindung zwischen den beiden betroffenen Kammern einstellen.

Gattungsgemäße orthopädietechnische Einrichtungen verfügen folglich über ein Hydrauliksystem mit beweglichen Teilen. Die Hydraulikflüssigkeit muss daher unterschiedliche Eigenschaften aufweisen und unterschiedliche Aufgaben erfüllen. Einerseits sollte die Viskosität der Hydraulikflüssigkeit sich beispielsweise bei verändernder Temperatur möglichst wenig verändern. Ob die orthopädietechnische Einrichtung beispielsweise im Winter bei Temperaturen unter dem Gefrierpunkt von Wasser oder im Sommer bei beispielsweise 30 °C verwendet wird, sollte die Viskosität der Hydraulikflüssigkeit möglichst wenig beeinflussen. Die Viskosität ist entscheidend für die Fließeigenschaften der Hydraulikflüssigkeit. Je höher die Viskosität ist, desto zähflüssiger und wenig fließfähig ist die Hydraulikflüssigkeit. Die Fließfähigkeit beeinflusst dabei selbstverständlich stark, wie schnell oder einfach die Hydraulikflüssigkeit durch das Hydrauliksystem und insbesondere durch das wenigstens eine Ventil hindurch fließen kann. Dadurch beeinflusst sie auch den Strömungswiderstand und damit die Dämpfung. Eine Hydraulikflüssigkeit, deren Viskosität sich in Abhängigkeit von der Temperatur stark ändert, hätte folglich zur Folge, dass der Dämpfungswiderstand, der bei gegebener Ventilstellung durch das Hydrauliksystem aufgebracht wird, temperaturabhängig wird, insbesondere bei laminarer Drosselung. Dies ist nicht gewünscht und würde den Komfort der orthopädietechnischen Einrichtung beeinträchtigen oder sogar den Verlust der Stabilität der orthopädietechnischen Einrichtung bedeuten.

Gleichzeitig sollte eine Hydraulikflüssigkeit die beweglichen Teile, die mit der Hydraulikflüssigkeit in Kontakt kommen, schmieren, um den Bewegungswiderstand, also die Reibung, der beweglichen Teile aneinander möglichst klein zu halten.

Aus dem Stand der Technik ist es bekannt, unterschiedliche Öle als Hydraulikflüssigkeit zu verwenden. So werden beispielsweise einkomponentige und mehrkomponentige Silikonöle verwendet, die eine vergleichsweise geringe Temperaturabhängigkeit der Viskosität aufweisen. Mehrkomponentige Silikonöle sind dabei Mischungen aus mehreren einzelnen Silikonölen, wobei die Mischung an die jeweiligen Aufgaben und Anforderungen, die das Öl als Hydraulikflüssigkeit erfüllen muss, angepasst sind.

In anderen orthopädietechnischen Einrichtungen werden Mineralöle oder pflanzliche Öle verwendet, die auch als Bioöle bezeichnet werden. Der Anwendungsbereich von Bioölen ist dabei jedoch begrenzt, da sie eine sehr starke Temperaturabhängigkeit der Viskosität aufweisen. Sie verfügen jedoch über exzellente Schmiereigenschaften und ein sehr gutes Verschleiß- und Verdampfungsverhalten.

Der Erfindung liegt die Aufgabe zugrunde, die Eigenschaften der verwendeten Hydraulikflüssigkeit zu verbessern und insbesondere die nur geringe Temperaturabhängigkeit der Viskosität der Hydraulikflüssigkeit, die durch das Silikonöl erreicht wird, mit guten Schmiereigenschaften zu kombinieren.

Die Erfindung löst die gestellte Aufgabe durch eine orthopädietechnische Einrichtung gemäß dem Oberbegriff des Anspruchs 1, die sich dadurch auszeichnet, dass die Hydraulikflüssigkeit zusätzlich ein Zusatzöl aufweist, das wenigstens eine Estergruppe aufweist. Eine Estergruppe verfügt insbesondere über wenigstens ein doppelt gebundenes Sauerstoffatom. Zur Bildung der Estergruppe wird eine Säuregruppe, beispielsweise eine Carboxy-Gruppe (C-O-OH) mit einem Alkohol zu einem Ester umgesetzt. Alternativ kann beispielsweise eine Sulfonyl-Gruppe mit einem Alkohol umgesetzt werden.

Der Erfindung liegt die überraschende Erkenntnis zugrunde, dass eine Kombination von Silikonöl und einem Zusatzöl, das wenigstens eine Ester-Gruppe aufweist, die gewünschten Eigenschaften aufweist und auch in einem Hydrauliksystem einer orthopädietechnischen Einrichtung beibehält. Aus dem Stand der Technik ist bekannt, das Silikonöl und ein solches Zusatzöl, beispielsweise ein Bioöl, nicht dauerhaft gemischt werden können. Selbst wenn man eine Emulsion aus beiden Ölen herstellt, entmischt sich die so hergestellte Hydraulikflüssigkeit nach einer Weile und die Phasen trennen sich.

Untersuchungen haben ergeben, dass diese Trennung zwischen dem Silikonöl und dem Zusatzöl auch innerhalb des Hydrauliksystems der orthopädietechnischen Einrichtung stattfindet, überraschenderweise jedoch die Funktion nicht beeinträchtigt, sondern eher noch unterstützt. Versuche mit orthopädietechnischen Einrichtungen und den Ventilen, die in deren Hydrauliksystem verwendet werden, haben gezeigt, dass die beiden Öle entmischen können. Dabei setzt sich das Zusatzöl an den metallischen Oberflächen, die sich im Innern des Hydrauliksystems und insbesondere im Innern des Ventils befinden, ab. Es sorgt auf diese Weise für eine optimale Schmierung der verschiedenen Bauteile und für eine deutliche Verringerung der Reibung. Das Silikonöl hingegen lagert sich an den metallischen Oberflächen nur wenig oder sogar gar nicht ab, sondern fließt durch das Hydrauliksystem, wenn es durch äußere Kräfte dazu angeregt und gezwungen wird. Auf diese Weise kann die sehr gute und über einen großen Temperaturbereich kaum veränderbare Viskosität des Silikonöls nahezu optimal genutzt werden.

Während sich ein Großteil des Zusatzöls folglich an den Oberflächen im Innern des Hydrauliksystems anhaftet und ablagert und somit beim Fließen der Hydraulikflüssigkeit kaum in Bewegung gerät, ist der Hauptteil des Silikonöls für die Bewegung der Hydraulikflüssigkeit zuständig. Die Entmischung, die zunächst als Problem angesehen wurde und den Stand der Technik davon abhält, andere Öle zu einem Silikonöl hinzu zu mischen, hat folglich große Vorteile, die dafür sorgt, dass die beiden verschiedenen Öle ihre jeweiligen Vorteile und positiven Eigenschaften voll einbringen können.

Durch die doppelt gebundenen Sauerstoffatome der Estergruppen und ihre starke Elektronegativität kommt es zu einer negativen Partialladung am Sauerstoffatom. Diese führt zu einer verstärkten elektrischen Anziehungskraft zu Metallen und Kunststoffen, wodurch das Zusatzöl an diesen Oberflächen haftet. Außerdem kommt es durch die elektrischen Partialladungen wahrscheinlich zu Dipol-Dipol-Wechselwirkungen und wahrscheinlich zur Bildung von Wasserstoffbrückenbindungen, so dass das Zusatzöl eine Schicht, vorzugsweise eine laminare Schicht an der Oberfläche der Bauteile ausbildet und so nicht nur das Silikonöl, sondern insbesondere auch Wasser von diesen Oberflächen verdrängt. Dadurch bildet sich ein Schmierfilm an der Oberfläche insbesondere metallischer Bauteile
Vorzugsweise weist die Hydraulikflüssigkeit höchstens 25 % Zusatzöl, bevorzugt höchstens 20 % Zusatzöl, besonders bevorzugt höchstens 15 % Zusatzöl auf. Das Verhältnis von Zusatzöl zur Gesamtmenge an verwendetem Öl beträgt beispielsweise 2:26, 3:26 oder 4:26, von denen sich das Verhältnis von 3:26 als besonders vorteilhaft gezeigt hat.

Vorzugsweise weist das Zusatzöl eine Glykolester-Gruppe und/oder eine Tryglycerid-Gruppe auf. Besonders bevorzugt ist das Zusatzöl ein Bioöl.

Die Erfindung löst die gestellte Aufgabe zudem durch ein Verfahren zum Füllen eines Hydrauliksystems für eine orthopädietechnische Einrichtung der hier beschriebenen Art, wobei bei dem Verfahren das Hydrauliksystem zunächst mit dem Zusatzöl gespült und dann mit Silikonöl aufgefüllt wird.

Besonders bevorzugt wird bei dem Spülen des Hydrauliksystems mit Zusatzöl das Hydrauliksystem zunächst vollständig mit Zusatzöl gefüllt und das Zusatzöl dann wieder abgelassen, wobei ein Anteil Zusatzöl im Hydrauliksystem verbleibt. Besonders bevorzugt verbleibt das gesamte Zusatzöl oder zumindest nahezu das gesamte Zusatzöl, beispielsweise mindestens 90% bevorzugt mindestens 95 % des verwendeten Zusatzöl im Hydrauliksystem.

Vorzugsweise werden zumindest einige der beweglichen Teile des Hydrauliksystems, besonders bevorzugt alle beweglichen Teile des Hydrauliksystems vor der Montage des Hydrauliksystems mit Zusatzöl benetzt. Alternativ dazu werden zumindest einige der beweglichen Teile, bevorzugt alle beweglichen Teile des Hydrauliksystems mit Zusatzöl benetzt, nachdem das Hydrauliksystem montiert wurde, jedoch bevor eine Hydraulikflüssigkeit eingefüllt wird.

Vorzugsweise werden das Zusatzöl und das Silikonöl an unterschiedlichen Positionen in das Hydrauliksystem eingefüllt. Dazu verfügt das Hydrauliksystems über wenigstens zwei verschiedene Öffnungen, in die Hydraulikflüssigkeit eingefüllt werden kann. Durch die Verwendung mehrerer verschiedene Öffnungen für die unterschiedlichen Bestandteile des gemischten Hydraulikfluids können die beiden Bestandteile gleichzeitig eingeführt werden, ohne dass es nötig ist, die Mischung vorher herzustellen.

Vorzugsweise werden die verschiedenen Öle außerhalb des Hydrauliksystems gemischt und dann in das Hydrauliksystem eingefüllt. Dies kann über mehrere oder eine einzige Einfüll-Stelle geschehen. Vorzugsweise werden das Silikonöl und das Zusatzöl gravimetrisch, also nach Gewichtsanteilen, gemischt. Daher gelten die hier genannten Mischungsverhältnis vorzugsweise auch für Gewichtsprozent.

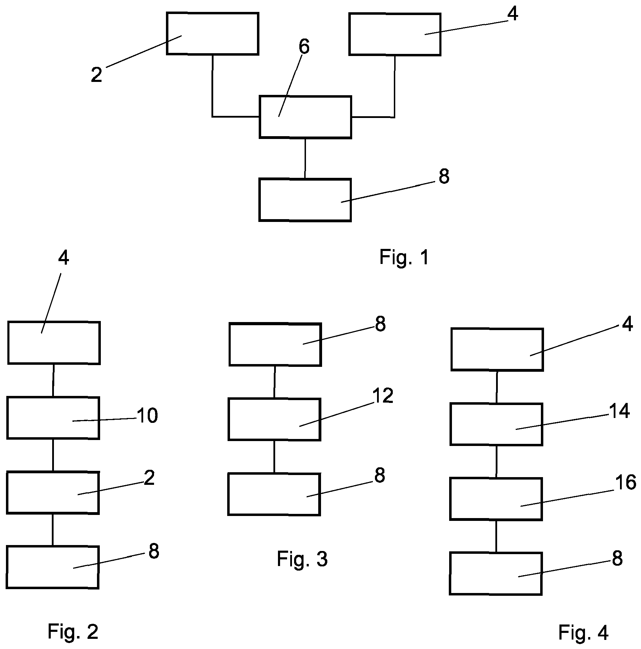

Mithilfe der beiliegenden Figuren werden nachfolgend einige Ausführungsbeispiele der vorliegenden Erfindung näher erläutert. Dabei zeigen die Figuren 1 bis 4 schematische unterschiedliche Verfahrensabläufe zum Befüllen eines Hydrauliksystems.

Das nicht erfindungsgemäße Verfahren gemäß Figur 1 beginnt mit dem Bereitstellen des Silikonöls 2 und dem Bereitstellen des Zusatzöls 4. Das Silikonöl und das Zusatzöl werden in einem Mischschritt 6 gemischt, wobei das gewünschte Verhältnis zwischen Zusatzöl und Silikonöl eingestellt wird. Anschließend erfolgt das Befüllen 8 des Hydrauliksystems mit den gemischten Ölen.

Figur 2 zeigt ein Verfahren gemäß der vorliegenden Erfindung. Es beginnt mit dem Bereitstellen des Zusatzöls 4, mit dem in einem Spülschritt 10 das Hydrauliksystem gespült wird. Zusatzöl an einer ersten Einfüll-Stelle in das Hydrauliksystem eingeführt, läuft durch möglichst das gesamte Hydrauliksystem hindurch und verlässt das Hydrauliksystem an einer Ablass-Stelle. Gegebenenfalls ist es ausreichend, nur Teile, beispielsweise die metallischen Bauteile und/oder die Bauteile, die zumindest teilweise eine mit dem Hydraulikfluids in kontaktkommende metallische Oberfläche haben, mit dem Zusatzöl zu spülen. Wichtig ist bei diesem Verfahren, dass in dem Spülschritt 10 das Zusatzöl lediglich durch das Hydrauliksystem oder einen Teil des Hydrauliksystems hindurchgeführt wird. Anschließend erfolgt das Bereitstellen des Silikonöls 2 und das spätere Befüllen 8, wobei beim Befüllen 8 das bereits mit dem Zusatzöl gespielte Hydrauliksystem mit dem Silikonöl befüllt wird. Ein Mischschritt 6 ist bei diesem Verfahren unnötig. Das Mischungsverhältnis zwischen dem Zusatzöl und dem Silikonöl, die sich nach Abschluss des Verfahrens gemäß Figur 2 im Innern des Hydrauliksystems befinden, bestimmt sich danach, welche Menge des Zusatzöls im Spülschritt 10 an den Oberflächen innerhalb des Hydrauliksystems haften bleibt und daher im Spülschritt 10 das Hydrauliksystem nicht wieder verlässt. Die einzelnen Oberflächen des Teils des Hydrauliksystems, dass im Spülschritt 10 mit dem Zusatzöl gespült wird, bleiben mit dem Zusatzöl benetzt, sodass eine gewisse Menge des Zusatzöls innerhalb des Hydrauliksystems verbleibt. Beim Befüllen 8 wird das Hydrauliksystem mit Silikonöl aufgefüllt.

Figur 3 zeigt eine Variante des Verfahrens. Dabei wird kein Spülschritt 10 durchgeführt, sondern das Hydrauliksystem wird beim ersten Befüllen 8 vollständig mit dem Zusatzöl gefüllt. Anschließend erfolgt ein Entleeren 12, bei dem das einem ersten Befüllen 8 in das Hydrauliksystem eingefüllt Zusatzöl wieder abgelassen wird. Anschließend erfolgt das zweite Befüllen 8, bei dem das auf diese Weise geleerte Hydrauliksystem mit Silikonöl aufgefüllt wird. Beim ersten Befüllen 8 und dem späteren Entleeren 12 verbleibt wie im Spülschritt 10 gemäß Figur 2 eine gewisse Menge des Zusatzöls innerhalb des Hydrauliksystems. Die mit der Hydraulikfluids in Kontakt kommenden Oberflächen des Hydrauliksystems bleiben mit dem Zusatzöl benetzt. Anschließend wird beim zweiten Befüllen 8 das Hydrauliksystem mit Silikonöl aufgefüllt.

Figur 4 zeigt ein weiteres nicht erfindungsgemäßes Verfahren. Dieses Verfahren beginnt mit dem Bereitstellen des Zusatzöls 4. Beim anschließenden Benetzen 14 werden zumindest einige der beweglichen Teile, vorzugsweise alle beweglichen Teile, des Hydrauliksystems mit dem Zusatzöl benetzt. Anschließend erfolgt die Montage 16 des Hydrauliksystems, bei der die benetzen beweglichen Teile verbaut werden. Auch auf diese Weise entsteht ein Hydrauliksystem, dessen Oberflächen, die mit dem Hydraulikfluids in Kontakt kommen, mit Zusatzöl benetzt sind. Anschließend erfolgt auch im Verfahren gemäß Figur 4 das Befüllen 8 des Hydrauliksystems mit dem Silikonöl.

### Bezugszeichenliste

- 2: Bereitstellen des Silikonöls
- 4: Bereitstellen des Zusatzöls
- 6: Mischschritt
- 8: Befüllen
- 10: Spülschritt
- 12: Entleeren
- 14: Benetzen
- 16: Montage

## Patentansprüche

1. Orthopädietechnische Einrichtung mit wenigstens einem Gelenk und einem Hydrauliksystem mit wenigstens einem Ventil, wobei das Hydrauliksystem mit einer Hydraulikflüssigkeit gefüllt ist, die ein Silikonöl aufweist,
**dadurch gekennzeichnet, dass**
die Hydraulikflüssigkeit zusätzlich ein Zusatzöl aufweist, das wenigstens eine Estergruppe aufweist.

2. Orthopädietechnische Einrichtung nach Anspruch 1, **dadurch gekennzeichnet, dass** die Hydraulikflüssigkeit höchstens 25 % Zusatzöl, bevorzugt höchstens 20 % Zusatzöl, besonders bevorzugt höchstens 15 % Zusatzöl aufweist.

3. Orthopädietechnische Einrichtung nach Anspruch 1 oder 2, **dadurch gekennzeichnet, dass** das Zusatzöl eine Glykolester-Gruppe und/oder eine Tryglycerid-Gruppe aufweist.

4. Orthopädietechnische Einrichtung nach einem der vorstehenden Ansprüche, **dadurch gekennzeichnet, dass** das Zusatzöl wenigstens ein Bioöl aufweist, bevorzugt wenigstens ein Bioöl ist.

5. Verfahren zum Füllen eines Hydrauliksystems für eine orthopädietechnische Einrichtung nach einem der vorstehenden Ansprüche, wobei bei dem Verfahren das Hydrauliksystem zunächst mit Zusatzöl gespült (10) und dann mit Silikonöl aufgefüllt wird (8), wobei das Zusatzöl wenigstens eine Estergruppe aufweist.

6. Verfahren nach Anspruch 5, **dadurch gekennzeichnet, dass** beim Spülen mit Zusatzöl das Hydrauliksystem zunächst vollständig mit Zusatzöl gefüllt (8) und das Zusatzöl dann wieder abgelassen (12) wird, wobei ein Anteil Zusatzöl im Hydrauliksystem verbleibt.

## Claims

1. An orthopedic device with at least one joint and a hydraulic system with at least one valve, the hydraulic system being filled with a hydraulic fluid, which contains a silicone oil,
**characterized in that**
the hydraulic fluid additionally comprises an additive oil, which contains at least one ester group.

2. The orthopedic device according to claim 1, **characterized in that** the hydraulic fluid comprises at most 25% additive oil, preferably at most 20% additive oil, especially preferably at most 15% additive oil.

3. The orthopedic device according to claim 1 or 2, **characterized in that** the additive oil contains a glycol ester group and/or a triglyceride group.

4. The orthopedic device according to one of the preceding claims, **characterized in that** the additive oil comprises at least one bio-oil; preferably, it is at least one bio-oil.

5. A method for filling a hydraulic system for an orthopedic device according to one of the preceding claims, wherein the hydraulic system is initially flushed (10) with additive oil and then filled (8) with silicone oil during the method, wherein the additive oil contains at least one ester group.

6. The method according to claim 5, **characterized in that**, during the flushing of the hydraulic system with additional oil, the hydraulic system is initially completely filled (8) with additive oil and the additive oil is then drained (12) again, wherein a proportion of additive oil remains in the hydraulic system.

## Revendications

1. Dispositif orthopédique comprenant au moins une articulation et un système hydraulique avec au moins une vanne, le système hydraulique étant rempli d'un liquide hydraulique qui comprend une huile à base de silicone,
**caractérisé en ce que**
le liquide hydraulique comprend en supplément une huile additionnelle qui comprend au moins un groupe ester.

2. Dispositif orthopédique selon la revendication 1,
**caractérisé en ce que** le liquide hydraulique comprend au maximum 25 % d'huile additionnelle, de préférence au maximum 20 % d'huile additionnelle, de manière particulièrement préférée au maximum 15 % d'huile additionnelle.

3. Dispositif orthopédique selon la revendication 1 ou 2,
**caractérisé en ce que** l'huile additionnelle comprend un groupe ester glycolique et/ou un groupe triglycéride.

4. Dispositif orthopédique selon l'une des revendications précédentes,
**caractérisé en ce que** l'huile additionnelle comprend au moins une huile biologique, de préférence est au moins une huile biologique.

5. Procédé de remplissage d'un système hydraulique pour un dispositif orthopédique selon l'une des revendications précédentes, procédé dans lequel le système hydraulique est d'abord rincé (10) avec de l'huile additionnelle, puis est rempli (8) d'huile à base de silicone,
l'huile additionnelle comprenant au moins un groupe ester.

6. Procédé selon la revendication 5,
**caractérisé en ce que**, lors du rinçage avec de l'huile additionnelle, le système hydraulique est d'abord entièrement rempli (8) d'huile additionnelle, puis l'huile additionnelle est évacuée (12), une partie de l'huile additionnelle restant dans le système hydraulique.
